# EUROPEAN PATENT SPECIFICATION

(11) **EP 1 028 750 B1**
(45) Date of publication and mention of the grant of the patent: **01.02.2006**
(21) Application number: 97909341.6
(22) Date of filing: 15.09.1997
(51) Int. Cl.: A61K 39/295

(54) **Method for preparing multivalent vaccines**
Verfahren zur Herstellung multivalenter Impfstoffe
Procédé de preparation de vaccins multivalents

(43) Date of publication of application: 23.08.2000
(73) Proprietor: Sanofi Pasteur MSD, 69007 Lyon (FR)
(72) Inventor: ARMINJON, François, F-69300 Caluire (FR); CARTIER, Jean-René, F-69005 Lyon (FR); LENTSCH-GRAF, Sandrine, F-69004 Lyon (FR); MARCHAL, Laurent, F-68100 Mulhouse (FR)
(74) Representative: Domenego, Bertrand
(86) International application number: PCT/EP1997/005378
(87) International publication number: WO 1999/013906

(56) References cited:
- WO-A-93/24148
- WO-A-98/00167
- KATZ S.: "ASSETS AND LIABILITIES OF COMBINED VACCINES" REP. PEDIATR. INFECT.DIS., vol. 4, no. 1, 1994, page 4 XP002065101
- GOLD R. AND AL.: "Safety and immunogenicity of Haemophilus influenzae vaccine (tetanus toxoid conjugate) administered concurrently or combined with diphtheria and tetanus toxoids, pertussis vaccine and inactivated poliomyelitis vaccine to healthy infants at two, four and six months of age" PEDIATR.INFECT.DIS.J., vol. 13, no. 5, 1994, pages 348-355, XP002065102 cited in the application

## Description

### BACKGROUND OF THE INVENTION

Infectious diseases remain a threat to human health despite decades of vaccine research. Combination vaccines which provide protection against multiple pathogens are very desirable to minimize the number of immunizations required to confer protection against multiple pathogens. The well documented phenomenon of antigenic competition complicates the development of multi-component vaccines. Antigenic competition refers to the observation that administering multiple antigens often results in a diminished response to certain antigens relative to the immune response observed when such antigens are administered individually.

The multiple pathogens against which the vaccines of the present invention provides protection are discussed in the context of the diseases they cause and the antigens from the pathogens which can be used in formulation of the present invention.

Whooping cough or pertussis is a severe, highly contagious upper respiratory tract infection caused by *Bordetella pertussis.* The World Health Organization estimates that there are 60 million cases of pertussis per year and 0.5 to 1 million associated deaths (ref. 1. Throughout this specification, various references are referred to in parenthesis to more fully describe the state of the art to which this invention pertains. Full bibliographic information for each citation is found at the end of the specification, immediately following the claims. The disclosures of these references are hereby incorporated by reference into the present disclosure). In unvaccinated populations, a pertussis incidence rate as high as 80% has been observed in children under 5 years old (ref. 2). Although pertussis is generally considered to be a childhood disease, there is increasing evidence of clinical and asymptomatic disease in adolescents and adults (refs. 3, 4, and 5).

The introduction of whole-cell vaccines composed of chemically- and heat-inactivated *B. pertussis* organisms in the 1940's was responsible for a dramatic reduction in the incidence of whooping cough caused by *B. pertussis.* The efficacy rates for whole-cell vaccines have been estimated at up to 95% depending on case definition (ref. 6). While infection with B. *pertussis* confers life-long immunity, there is increasing evidence for waning protection after immunization with whole-cell vaccines (ref. 3). Several reports citing a relationship between whole-cell pertussis vaccination, reactogenicity and serious side-effects led to a decline in vaccine acceptance and consequent renewed epidemics (ref. 7). More recently, defined component pertussis vaccines have been developed.

### Antigens for Defined Pertussis Vaccines

Various acellular pertussis vaccines have been developed and include the *Bordetella* pertussis antigens, Pertussis Toxin (PT), Filamentous haemagglutinin (FHA), the 69kDa outer membrane protein (pertactin) and fimbrial agglutinogens. PT and FHA are included in the formulations of the present invention and are described in more detail below.

### Pertussis Toxin

Pertussis toxin is an exotoxin which is a member of the A/B family of bacterial toxins with ADP-ribosyltranserase activity (ref. 8). The A-moiety of these toxins exhibit the ADP-ribosyltransferase activity and the B-moiety mediates binding of the toxin to host cell receptors and the translocation of A to its site of action. PT also facilitates the adherence *of B*. *pertussis* to ciliated epithelial cells (ref. 9) and also plays a role in the invasion of macrophages by B. *pertussis* (ref. 10).

All acellular pertussis vaccines have included PT, which has been proposed as a major virulence factor and protective antigen (ref. 11, 12). Natural infection with B. pertussis generates both humoral and cell-mediated responses to PT (refs. 13 to 17). Infants have transplacentally-derived anti-PT antibodies (refs. 16, 18) and human colostrum containing anti-PT antibodies was effective in the passive protection of mice against aerosol infection (ref. 19). A cell-mediated immune (CMI) response to PT subunits has been demonstrated after immunization with an acellular vaccine (ref. 20) and a CMI response to PT was generated after whole-cell vaccination (ref. 13). Chemically-inactivated PT in whole-cell or component vaccines is protective in animal models and in humans (ref. 21). Furthermore, monoclonal antibodies specific for subunit S1 protect against *B. pertussis* infection (refs. 22 and 23).

The main pathophysiological effects of PT are due to its ADP-ribosyltransferase activity. PT catalyses the transfer of ADP-ribose from NAD to the Gi guanine nucleotide-binding protein, thus disrupting the cellular adenylate cyclase regulatory system (ref. 24). PT also prevents the migration of macrophages and lymphocytes to sites of inflammation and interferes with the neutrophil-mediated phagocytosis and killing of bacteria (ref. 25). A number of *in vitro* and *in vivo* assays have been used to asses the enzymatic activity of S1 and/or PT, including the ADP-ribosylation of bovine transducin (ref. 26), the Chinese hamster ovary (CHO) cell clustering assay (ref. 27), histamine sensitization (ref. 28), leukocytosis, and NAD glycohydrolase. When exposed to PT, CHO cells develop a characteristic clustered morphology. This phenomenon is dependent upon the binding of PT, and subsequent translocation and ADP-ribosyltransferase activity of S1 and thus the CHO cell clustering assay is widely used to test the integrity and toxicity of PT holotoxins.

PT must be detoxified prior to its inclusion in a vaccine formulation. Several techniques of chemical detoxification have been described, including inactivation with formalin (ref. 46), glutaraldehyde (ref. 52), hydrogen peroxide (ref. 53) and tetranitromethane (ref. 54). Alternatively, mutant strains of *B. pertussis* or recombinant host cells expressing genetically detoxified PT may be used to prepare enzymatically inactive PT which retains immuological activity.

### Filamentous Haemagglutinin

Filamentous haemagglutinin is a large (220 kDa) non-toxic polypeptide which mediates attachment of *B*. *pertussis* to ciliated cells of the upper respiratory tract during bacterial colonization (refs. 9, 29). Natural infection induces anti-FHA antibodies and cell mediated immunity (Refs. 13, 15, 17, 30 and 31). Anti-FHA antibodies are found in human colostrum and are also transmitted transplacentally (refs. 17, 18 and 19). Vaccination with whole-cell or acellular pertussis vaccines generates anti-FHA antibodies and acellular vaccines containing FHA also induce a CMI response to FHA (refs. 20, 32). FHA is a protective antigen in a mouse respiratory challenge model after active or passive immunization (refs. 33, 34). However, alone FHA does not protect in the mouse intracerebral challenge potency assay (ref. 28).

### Acellular Pertussis Vaccines

The first acellular pertussis vaccine developed was the two-component PT + FHA vaccine (JNIH 6) of Sato et al. (ref. 46). This vaccine was prepared by co-purification of PT and FHA antigens from the culture supernatant of *B. pertussis* strain Tohama, followed by formalin toxoiding. Acellular vaccines from various manufacturers and of various compositions have been used successfully to immunize Japanese children against whopping cough since 1981 resulting in a dramatic decrease in incidence of disease (ref. 47). The JNIH 6 vaccine and mono-component PT toxoid vaccine (JNIH 7) were tested in a large clinical trial in Sweden in 1986. Initial results indicated lower efficacy than the reported efficacy of a whole-cell vaccine, but follow-up studies have shown it to be more effective against milder disease diagnosed by serological methods (refs. 48, 49, 50, 51). However, there was evidence for reversion to toxicity of formalin-inactivated PT in these vaccines. These vaccines were also found to protect against disease rather than infection.

A number of new acellular pertussis vaccines are currently being assessed which include combinations of PT, FHA, and/or 69 kDa Outer Membrane Protein (Pertactin), and/or fimbrael agglutinogens.

### Tetanus

Tetanus is an acute infection caused by *Clostridium tetani.* The disease is characterized by severe, painful muscle contractions, accompanied by hypersensitivity, hyperreflexia and increased autonomic stimulation of the affected body part(s). Mild stimuli may cause severe reflex muscle spasms. Fever due to extreme muscle spasm may be present. Tetanus may be generalized, involving the face, neck, abdomen and trunk or localized to a specific body part (injury site). Involvement of the masseter muscle of the face results in trismus or lockjaw giving rise to the classical facial expression known as "risus sardonicus" (ref. 78).

*C. tetani* exists as a nonpathogenic organism in the gut of humans and animals. The organism is also found in soil contaminated by feces and may survive in soil for years as infectious spores (ref. 79).

Tetanus results from the anaerobic growth of *C*. *tetani* and neurotoxin production in contaminated wounds. Infection is caused by the introduction of materials contaminated by organisms or spores into tissue. The most common scenario is infection through a penetrating injury. However, in many cases no history of injury is obtainable. The presence of necrotic or ischemic tissue facilitates the growth of the bacillus (ref. 78).

Prevention of infection is by vaccination and by good wound care including careful cleaning and debridement of devitalized tissues. Individuals with contaminated wounds and who have failed series should be given both tetanus vaccine and tetanus immune globulin.

Treatment of the syndrome is mainly supportive and may include respiratory support, administration of tetanus antitoxin and careful cleaning of infected wounds. Despite modem medical care the case fatality rates still run as high as 30 to 90% (ref. 79). This is particularly true tin the elderly. Natural infection does not always produce immunity from further infection.

Prevention of infection by vaccination is the most effective method of controlling the disease. Since the introduction of universal vaccination, tetanus has become extremely rare in developed countries. Cases occur almost exclusively in individuals who failed to complete their series of vaccinations or who have not received appropriate booster doses. Individuals should receive a booster dose once every ten years.

### Diphtheria

Diphtheria is an acute infection caused by the bacteria *Corynebacterium diphtheriae.* The main site of infection is the upper respiratory tract (nose, pharynx, larynx and trachea) (ref. 80). The characteristic lesion, a result of the bacterial cytotoxin, are patches of greyish pseudomembrane surrounded by inflammation. This is accompanied by cervical lymphadenopthy, swelling and edema of the throat. In severe cases the swelling may progress to the point of obstruction (laryngeal diphtheria). Other complications include myocarditis, central nervous system effects (cranial, motor and sensory neuropathies such as ascending paralysis), and thrombocytopenia. Other mucosal membranes may be less frequently affected. The clinical presentation may vary from asymptomatic infection to fulminant multisystem, and death (ref. 79). Cutaneous and would infections with diphtheria are common in the tropics and have been frequently reported in the U.S. indigent population. The only reservoir for C. *diphiheriae* is man (ref. 79).

A presumptive diagnosis may be made on clinical observation of the characteristic lesions but should be confirmed by bacterial examination of the lesions. If there is a strong clinical suspicion of diphtheria, treatment should be initiated immediately with antibiotics (penicillin or erythromycin) and diphtheria antitoxin, even if the diagnosis is not confirmed. Mortality increases the longer one waits after the onset of clinical symptoms (ref. 80). The case fatality rate ranges from five to ten per cent despite modem medical care (ref. 79) and occurs mainly in the very young and in the elderly. Natural infection does not always produce immunity from further infection (ref. 80).

Transmission is by direct contact with secretions or discharges from an infected individual. Individuals are contagious as long as bacteria are observed in the secretions. This may last up to four weeks after infection. Transmission may also occur with infected fomites (ref. 79). Strict isolation of cases is recommended.

Rarely individuals may become carriers and shed organisms up to six months after infection. Unimmunized carriers should be promptly vaccinated with the full series. Treatment with antibiotics eliminates carriage and infectiousness of cases in 4 days (ref. 80).

### Poliomyelitis

Both inactivated (IPV) and live attenuated (OPV) poliovirus vaccines have been effective in controlling poliomyelitis world wide. A combined DPT-IPV vaccine is currently licensed in Europe and in Canada and has been shown to be safe and effective in millions of children worldwide.

### Haemophilus influenzae type b

Prior to the availability of effective vaccines, *Haemophilus influenzae* type b (Hib) was a major cause of meningitis invasive bloodborne infections in young children and was the main cause of meningitis in the first 2 years of life (ref. 81). Approximately 10% of *Haemophilus influenzae* meningitis victims die despite medical care. Permanent sequelae are common in survivors. Immunization against *Haemophilus injluenzae* began in Canada in 1987 with a polysaccharide vaccine (polyribose ribitol phosphate [PRP]). Improved immunogenicity was achieved in children 18 months of age and older with the introduction in 1988 of a vaccine consisting of PRP conjugated to diphtheria toxoid (PRP-D) Since 1992, infant immunization has been possible with the licensure of PRP conjugate vaccines immunogenic in infants under 1 year of age (PRP conjugated with tetanus toxoid or PRP-T). Use of these *Haemophilus influenzae* conjugate vaccines has been associated with a dramatic decrease in the incidence of invasive *Haemophilus* infection in Canada and elsewhere (ref. 82). Two Canadian clinical studies involving nearly 900 children in British Columbia and Alberta demonstrated that lyophilized PRP-T may be reconstituted with DPT (COMBIPACK) (ref. 83) or with DPT-Polio Adsorbed (PENTA™) (ref. 84) in addition to the usual saline diluent. Clinical studies involving more than 100,000 children around the world have demonstrated the efficacy of lyophilized PRP-T (ActHib™). Over 90% achieve anti-PRP levels considered to be protective (≥0.15 µg/ml) after 3 doses of PRP-T starting at 2 months or after a single does of PRP-T given after 12 months of age. The proportion achieving levels indicative of long term protection (>1.0 µg/ml) varies from 70 to 100% depending on the study. Millions of doses of PRP-T have been sold in the United States, Canada and Europe since 1992. Breakthrough cases of invasive haemophilus infection after vaccination with PRP-T are rare and may be associated with diseases such as immunodeficiency (ref. 85).

### Combination Vaccines

Although there are many actual and potential benefits of vaccines that combine antigens to confer protection against multiple pathogens, these combinations may have a detrimental effect on the immunogenicity of the individual components. Combinations of diphtheria and tetanus toxoids with whole cell pertussis vaccine (DTP) have been available for over 50 years and the antibody response to the combination is superior to that of the individual components, perhaps as a result of tha adjuvant effect of the whole cell pertussis vaccine. DTP combinations that also include inactivated poliovirus vaccine are licensed in many jurisdictions, although the antibody response to the pertussis antigens may be diminished by this combination (refs 69 to 71). The effect of combining DTP vaccines with Hib conjugate vaccine have been variable. Studies with a French DTP and PRPT demonstrated similar safety but a decreased antibody response to PRP (ref. 72 to 73) whereas studies with a Canadian DTP and PRPT showed no effect on the PRP response but lower pertussis agglutinogens and increased injection site tenderness in the combined immunization group (refs 74,75).

Data are now becoming available on the effect of combining acellular pertussis/diphtheria/tetanus (APDT) vaccines with Hib conjugate vaccine. In two month old infants given three doses of an acellular pertussis-diphtheria-tetanus vaccine combined with a Hib conjugate vaccine, the antibody response to PRP was significantly lower than in the group given separate injections on the same day (ref. 76). Similar results were reported with another acellular pertussis-diphtheria-tetanus vaccine combined with PRPT given for the first three doses (ref. 77).

In contrast to other reported studies, children immunized with the combined vaccine had a superior antibody response to PRP, diphtheria, and several of the pertussis antigens when compared to children given PRP at a separate visit. A liquid combination of diphteria, tetanus, perntssis (DTP) and Haemophilus influenzae type b (PRP-T) was safe and at least as immunogenic as the lyophilized preparation which corresponds to the reconstitution of Hib with DTP (ref. 86). There may be several reasons for the equivalent or better immunogenicity for these vaccines when given as a combined injection rather than the decreased immunogenicity reported with other products. All acellular pertussis vaccines are not identical in their antigenic content, method of toxoiding, adjuvant or preservative. However, increased immunogenicity has been reported with acellular pertussis vaccines containing PT, FHA, and 69K (ref. 77) and containing PT, FHA, 69K and fimbriae (ref. 76).

A five component APDT vaccine was found to have a protective efficacy of 85% (95% CI 81/89) in a phase III clinical trial recently completed in Sweden under the auspices of the National Institutes of Health (ref. 78).

More recently WO98/00167 describes a multivalent immunogenic composition combining acellular pertussis / diphtheria / tetanus (APDT) vaccines with Hib conjugate vaccine and polio vaccine. This multivalent immunogenic composition may further comprise an aluminium salt as adjuvant.
The multivalent immunogenic compositions are prepared just by mixing single antigenic preparations in the presence of adjuvant. WO98/00167 shall be taken into account according to A.54(3)EPC.

WO93/24148 describes multivalent immunogenic compositions, all of them containing the Hepatitis B surface antigen (HBsAg) component. These multivalent compositions are prepared by mixing aluminium phosphate adsorbed HBsAg, with one or more aluminium hydroxide (or phosphate) adsorbed antigens.

Current commercially-available combination vaccines may not contain appropriate formulations of appropriate antigens in appropriate immunogenic forms to achieve a desired level of efficacy in a susceptible human population.

It would be desirable to provide efficacious combination vaccines comprising acellular pertussis components together with selected relative amounts of selected antigens such as tetanus toxoid, diphtheria toxoid, *Haemophilus influenza* type B polysaccharide conjugate, poliovirus, and/or Hepatitis B Surface Ag. It would be highly desirable to develop a multivalent vaccine against diseases caused by infection by *Bordetella pertussis, Corynebacterium diphtheriae, Clostridium tetanus, Haemophilus influenzae,* poliovirus and hepatitis B virus. However, in order for such combination vaccines to be effective at achieving the criterion of seroprotection for each individual antigenic component, significant challenges in the form of antigenic competition and interference phenomena must be overcome. The present invention overcomes the limitations of the prior art and solves the problems of antigenic competition and interference by providing formulations of up to nine separate antigens designed to elicit seroprotection for up to six different infectious diseases.

### SUMMARY OF THE INVENTION

The present invention is directed towards combination or multivalent vaccines containing a plurality of vaccine components suitable for the prevention, amelioration or treatment of multiple disease states which meet the criterion for seroprotection for each of said vaccine components, and methods of use thereof. In accordance with the invention, there is provided a multivalent immunogenic composition for conferring protection in a host against disease caused by infection by *Bordetella pertussis, Corynebacterium diphtheriae, Clostridium tetanus,* poliovirus, hepatitis B virus and *Haemophilus trifluenzae.*

According to one embodiment of the object of the invention, there is provided a method for preparing a vaccine composition comprising:
(a) pertussis toxoid and filamentous haemagglutinin in purified form,
(b) tetanus toxoid,
(c) diphtheria toxoid,
(d) inactivated polio virus,
(e) an *Haemophilus influenzae* type B polysaccharide conjugate,
(f) an Hepatitis B surface antigen and
(g) an aluminium salt,

said method comprising the following steps :
(1) preparing an acidified aluminium hydroxide gel suspension at room temperature;
(2) adding successively diphtheria toxoid and tetanus toxoid to the gel suspension ;
(3) adding pertussis toxoid and filamentous haemagglutinin, each adsorbed separately onto aluminium salts, to the mixture obtained in (2), followed by a at least 30 minute agitation and a further overnight rest ;
(4) adding to the mixture obtained in (3) carbonate buffers in medium 199 and adjusting the pH to about 7,0 to 7,2;
(5) introducing inactivated poliovirus into the mixture obtained in (4) and then adjusting the pH to a value ranging from 6,8 to 7,0 ;
(6) adding Hepatitis B surface antigen previously adsorbed onto aluminium salts, to the mixture obtained in (5) followed by a at least 30 minute agitation; and
(7) adding a solution of an *Haemophilus influenzae* type B polysaccharide conjugate previously prepared in a phosphate buffer and a Tris-sucrose buffer to the mixture obtained in (6), followed by a at least 30 minute agitation.

According to a further embodiment of the object of the invention, there is provided a method for preparing a vaccine composition comprising:
(a) pertussis toxoid and filamentous haemagglutinin in purified form,
(b) tetanus toxoid,
(c) diphtheria toxoid,
(d) inactivated polio virus,
(e) an *Haemophilus influenzae* type B polysaccharide conjugate,
(f) an Hepatitis B surface antigen and
(g) an aluminium salt

said method comprising the following steps :
(1) preparing an acidified aluminium hydroxide gel suspension at room temperature;
(2) adding diphtheria toxoid and tetanus toxoid to the gel suspension;
(3) adding pertussis toxoid and filamentous haemagglutinin, each adsorbed separately onto aluminium salts, to the mixture obtained in (2), followed by a at least 30 minute agitation and a further overnight rest;
(4) adding to the mixture obtained in (3) carbonate buffers in medium 199 and adjusting the pH to about 7,0 to 7,2.
(5) introducing inactivated poliovirus into the mixture obtained in (4) and then adjusting the pH to a value ranging from 6,8 to 7,0;
(6) adding a solution of an *Haemophilus influenzae* type B polysaccharide conjugate prepared in a phosphate buffer and a Tris-sucrose buffer to the mixture obtained in (5) followed by a at least 30 minute agitation.

The vaccine compositions obtained by such methods may contain about 5 to about 30 ug of pertussis toxoid, about 5 to about 30 ug of filmentous hemagluttinin, about 5 to about 50 LF of diphtheria toxoid, about 5 to about 50 LF of tetanus toxoid, about 5 to about 20 ug of Hib conjugate and about 1 to about 10 ug HBsAg, all in combination with IPV.

The inactivated poliovirus employed in the immunogenic composition of the invention generally comprises a mixture of inactivated poliovirus types 1, 2 and 3. In one formulation, such mixtures of inactivated poliovirus types may comprise:
from about 20 to about 50 antigen units of poliovirus type 1;
from about 4 to about 10 antigen units of poliovirus type 2; and
from about 8 to about 40 antigen units of poliovirus type 3 in a single human dose.

The conjugate molecule may comprise a conjugate of suitable carrier protein, for example, tetanus toxoid or diphtheria toxoid, and polyribose ribitol phosphate (PRP) of Haemophilus influenzae type B. In a preferred formulation; the conjugate is employed in the form of about 10 ug of PRP conjugated to about 20 ug of tetanus toxoid.

In another aspect of the invention, there is provided a method of immunizing a host against multiple diseases, comprising administering to the host, which may be human, an immunoeffective amount of the immunogenic composition or vaccine as provided herein.

Advantages of the present invention include a multivalent vaccine which can confer protection against a range of common diseases in a safe and efficaceous manner. The ability to provide a single vaccination against multiple diseases without interference between the immunogenic responses to the various immunogens is beneficial .

The use of the multivalent vaccine of the present invention will reduce the number of injections and vaccination visits necessary for immunization. This will be especially useful and advantageous for childhood immunization.

### DETAILED DESCRIPTION OF THE INVENTION

Pertussis toxin (PT) (including genetically detoxified analogs thereof, as described in, for example, Klein *et al.,* U.S. Patent No. 5, 085, 862) may be produced by a variety of methods. For example, PT may be isolated from the culture supernatant of a B. pertussis strain using conventional methods such as that described by Sakura (ref. 55). PT is isolated by first absorbing culture supernatant onto a colun containing the dye-ligand gel matrix Affi-Gel Blue (Bio-Rad Laboratories. Richmond, CA). PT is eluted from this column by high salt, such as, 0.75 M magnesium chloride, and, after removing the salt, is passed through a column of fetuin-Sepharose affinity matrix composed of fetuin linked to cyanogen-bromide activated Sepharose. PT is eluted from the fetuin column using 4M magnesium salt.

Alternatively, the method of Irons *et al.* (ref 56) may be used. Culture supernatant is absorbed onto a CNBr-activatged Sepharose 4B colum to which haptogobin is first covalently bound. The PT binds to the adsorbent at pH 6.5 and is eluted from the column using 0.1M Tris/0.5M NaCl buffer by a stepwiee change to pH 10.0.

Alternatively, the method described in U.S. Patent No. 4,705,686 granted to Scott *et al*. on November 10, 1987 and incorporated herein by reference thereto may be used. In this method culture supernatants or cellular extracts of *B. pertussis* are passed through a column of an anion exchange resin of sufficient capacity to adsorb endotoxin but permit *Bordetella* antigens to flow through or otherwise be separated from the endotoxin.

Alternatively, PT may be purified by using perlite chromatography, as described in EP Patent No. 336 736.

### Detoxification of PT

PT is detoxified to remove undesired activities which could cause side reactions of the final vaccine. Any of a variety of conventional chemical detoxification methods can be used, such as treatment with formaldehyde, hydrogen peroxide, tetranitro-methane, or glutaraldehyde.

For example, PT can be detoxified with glutaraldehyde using a modification of the procedure described in Munoz *et al.* (ref. 57). In this detoxification process purified PT is incubated in a solution containing 0.01 M phosphate buffered saline. The solution is made 0.05\ with glutaraldehyde and the mixture is incubated at room temperature for two hours, and then made 0.02 M with L-lysine. The mixture is further incubated for two hours at room temperature and then dialyzed for two days against 0.01 M PBS. In a particular embodiment, the detoxification process of EP Patent No. 336 736 may be used. Briefly, PT may be detoxified with glutaraldehyde as follows:

Purified PT in 75mM potassium phosphate at pH 8.0 containing 0.22M sodium chloride is diluted with an equal volume of glycerol to protein concentrations of approximately 50 to 400 ug/ml. The solution is heated to 37°C and detoxified by the addition of glutaraldehyde to a final concentration of 0.5% (w/v). The mixture is kept at 37°C for 4 hrs and then aspartic acid (1.5 M) is added to a final concentration of 0.25 M. The mixture is incubated at room temperature for 1 hour and then diafiltered with 10 volumes of 10 mM potassium phosphate at pH 8.0 containing 0.15M sodium chloride and 5t glycerol to reduce the glycerol and to remove the glutaraldehyde. The PT toxoid is sterile-filtered through a 0.2 uM membrane.

If recombinant techniques are used to prepare a PT mutant molecule which shows no or little toxicity, for use as the toxoided molecule, chemical detoxification is not necessary.

### Purification of FHA

FHA may be purified from the culture supernatant essentially as described by Cowell *et al.* (ref. 58).. Growth promoters, such as methylated beta-cyclodextrins, may be used to increase the yield of FHA in culture supernatants. The culture supernatant is applied to a hydroxylapatite column. FHA is adsorbed onto the column, but PT is not. The column is extensively washed with Triton X-100 to remove endotoxin. FHA is then eluted using 0.5M NaCl in 0.1 M sodium phosphate and, if needed, passed through a fetuin-Sepharose column to remove residual PT. Additional purification can involve passage though a Sepharose CL-6B column.

Alternatively, FHA may be purified using monoclonal antibodies to the antigen, where the antibodies are affixed to a CNBr-activated affinity column (ref. 59).

Alternatively, FHA may be purified by using perlite chromatography as described in the above-mentioned EP 336 736.

### PT + FHA Vaccines

Such a vaccine could be prepared as described in EP 242 301 and in EP 242 302.

### Hib antigens

Such antigens can be based on the capsular polysaccharide (PRP) conjugated with a carrier protein, The polymer is a polymer of ribose, ribitol and phosphate. Tipically, the carrier protein is a diphteria or tetanus toxoid or an outer membran of N.meningitidis. Such conjugates are for example disclosed in EP 161,188, EP 208,375, EP 477,508, US 4,365,170 or US 4,673,574.

Such polysaccharide conjugates may be prepared by any known coupling technique as described for examples in WO 93/15760 or in patents cited in the previous sentence.

If one of the Hib antigens selected for the composition is a Capsular Polysaccharide Tetanus Toxoid Conjugate (PRP-T is an example) and if an aluminium salt (aluminium hydroxid is an example) is used in the composition, the Capsular Polysaccharide Tetanus Toxoid Conjugate is less stable and less immunogen than without aluminium salt. In such cases, these problems of stability and immunogenicity can be solved by using the technology described in WO96/37222. In brief, it consists in adding anions to the aluminium salts. Said anions can be phosphates, citrates. Phosphates can be provided by a monopotassium phosphate, dissodium phosphate solution. A combination of phosphates and carbonates (sodium carbonate, sodium bicarbonate) can be used as anions too.

### Polio vitus vaccine

The poliovirus component of the combination may be the Salk inactivated polio vaccine.

### Selected Multivalent Vaccine Formulations

In selected embodiments, the invention provides vaccines with the following characteristics, all of which may be administered by intramuscular injection:

One formulation comprises a combination of component pertussis vaccine combined with diphtheria and tetanus toxoids, inactivated poliovirus, Haemophilus influenza type B polysaccharide conjugate, and Hepatitis B Surface Ag and is termed Full liquid DTacP-IPV PRP-T-HBsAg.

Each 0.5 ml human dose of Full liquid DTacP-IPV-PRP-T-HBsAg was formulated to contain about:

| | |
|---|---|
| 25 µg | Pertussis toxoid (PT) |
| 25 µg | Filamentous haemagglutinin (FHA) |
| 30 LF | Diphtheria toxoid |
| 10 LF | Tetanus toxoid |
| 40 D | antigen units Poliovirus type 1 |
| 8D | antigen units Poliovirus type 2 |
| 32 D | antigen units Poliovirus type 3 |
| 10 µg | Haemophilus influenza type B polysaccharide covalently bound to |
| 20 µg | Tetanus protein |
| 5 µg | Hepatitis B Surface Ag |
| 20 µMoles | phosphates |
| 5 µMoles | carbonates |
| 0.125 ml | tris 50mMolaire buffer comprising saccharose in 42,5 % |
| 0.306 mg | Aluminum salts |

Another formulation, which is referred to herein as "DTaP-IPV-PRP~T/HBsAg" or the "dual chamber format", is comprised of a mixture of antigens in solution in the proximal chamber of a bypass syringe; the remaining components are provided in the distal chamber of the bypass syringe. The resulting composition is the same as that provided hereinabove with the difference that aluminium salts are present in a quantity of 0,356 mg.

### Vaccine Use

Immunogenic compositions and vaccines may be administered parenterally, by injection subcutaneously or intramuscularly. The immunogenic preparations and vaccines are administered in a manner compatible with the dosage formulation, and in such amount as will be therapeutically effective, immunogenic and protective. The quantity to be administered depends on the subject to be treated, including, for example, the capacity of the immune system of the individual to synthesize antibodies, and, if needed, to produce a cell-mediated immune response.

Suitable regimes for initial administration and booster doses are also variable, but may include an initial administration followed by subsequent administrations. The dosage may also depend on the route of administration and will vary according to the size of the host. Immunogenicity can be significantly improved if the antigens are coadministered with adjuvants, commonly used as 0.005 to 0.5 percent solution. Adjuvants enhance the immunogenicity of an antigen but are not necessarily immunogenic themselves.

Adjuvants may act by retaining the antigen locally near the site of administration to produce a depot effect facilitating a slow, sustained release of antigen to cells of the immune system. Adjuvants can also attract cells of the immune system to an antigen depot and stimulate such cells to elicit immune responses.

Immunostimulatory agents or adjuvants have been used for many years to improve the host immune responses to, for example, vaccines. Intrinsic adjuvants, such as lipopolysaccharides, normally are the components of the killed or attenuated bacteria used as vaccines. Extrinsic adjuvants are immunomodulators which are typically noncovalently linked to antigens and are formulated to enhance the host immune responses. Thus, adjuvants have been identified that enhance the immune response to antigens delivered parenterally. Some of these adjuvants are toxic, however, and can cause undesirable side effects, making them unsuitable for use in humans and many animals. Indeed, only aluminum hydroxide and aluminum phosphate (collectively commonly referred to as alum) are routinely used as adjuvants in human and veterinary vaccines. The efficacy of alum in increasing antibody responses to diphtheria and tetanus toxoids is well established.

Methods of protein biochemistry, fermentation and immunology used but not explicitly described in this disclosure and these Examples are amply reported in the scientific literature and are well within the ability of those skilled in the art.

### Example 1:

### Preparation of Full liquid formulation DTacP-IPV-PRB~T-HBsAg

A preferred formulation of a vaccine composition of the present invention comprises a liquid suspension of an immunoeffective amount of up to nine separate antigens, selected to elicit protection against as m.any as six infectious agents. This formulation, in which all of said antigens are present in solution for convenient administration to a human host, is designated or "Full liquid DtacP-IPV-PRP-T-HBs" or "full liquid" for short. The preferred method of manufacture of the full liquid formulation of the present invention is as follows.

An acidified aluminium hydroxide gel suspension is prepared by mixing at room temperature with pharmaceutical grade water. Successive additions of diphtheria toxoid (DT) and tetanus toxoid (TT) are then made to the gel suspension. The order of addition of these components is not critical, but the solution is preferably stirred for at least 30 minutes then allowed to settle for at least 30 minutes after the addition of each individual antigen component. Pertussis toxoid (PT) and filamentous haemagluttinin (FHA) are each adsorbed separately to aluminum salts and concentrated if necessary. These components are added to the mixture above, stirred for at least 30 minutes, and allowed to settle overnight.

At this point, carbonate buffers in Medium 199 are added, preferably through a 0.2µm filter, and either NaOH (2.5M) or acetic acid (10%) is added as needed to adjust the pH to about 7 to 7.2.

Next, IPV is introduced to an appropriate concentration with pharmaceutical grade water and introduced into the mixture, preferably through a 0.2 µm filter. The pH is then adjusted to a value ranging from 6.8 to 7.

Next, HBsAg, which has been previously adsorbed to aluminium salts, is added and stirred for at least 30 minutes.

Phosphate buffer, Tris-sucrose buffer and Water For Injection are added on *Haemophilus influenzae* type b polysaccharide conjugate (Hib) concentrate solution.

Finally, the buffered Hib solution is added to the other vaccine components, preferably to a 0.22µm filter, and stirred at least 30 minutes.

The resulting suspension obtained generally from the process set forth above is referred to as the full liquid bulk product. This bulk is then used to prepare the individual 0.5ml doses for use in clinical studies and vaccination procedures. Those skilled in the art will appreciate that the orders of addition of individual components, buffers used to dilute individual components, methods of addition and mixing, acids and bases used to adjust pH and mixing conditions can be modified without deviating from the spirit of the invention claimed herein.

### Example 2:

### Preparation of dual chamber formulation DtacP-IPV-PRP~T/HBsAg

Another formulation of a vaccine composition of the present invention comprises a liquid suspension of an immunoeffective amount of up to eight separate antigens, selected to elicit protection against as many as five infectious agents is present in a first, proximal chamber of a 1mL by-pass syringe and an immunoeffective amount of another antigen, selected to elicit protection against an additional infectious agent, is present in a second, distal chamber of said by-pass syringe. This formulation, in which all antigens are present in solution and in which certain of said antigens are disposed within the distal chamber of a bypass syringe and remaining antigen(s) are disposed within the proximal chamber of a bypass syringe is designated "Dual Chamber DtacP-IPV-PRP~T-HBs" or "dual chamber" for short.

One method of manufacture of the dual chamber formulation of the present invention entails a minor modification of the manufacturing method provided for the full liquid bulk in Example 1. All of the steps provided for manufacture of the full liquid bulk are the same, except that the step in which HBsAg is added to the mixture is omitted. The resulting solution is designated the DtacP-IPV-PRP~T bulk. 0.5mL of the DtacP-IPV-PRP~T bulk is disposed within the distal chamber of a 1.0 cc by-pass syringe. 0.5ml of an immunoeffective dose of HBsAg which has been previously adsorbed to aluminium salts is disposed within the proximal chamber of said by-pass syringe.

### Example 3:

### Clinical Trials

Clinical trials were performed in humans as described herein to establish the safety, nonreactogenicity and utility of the multivalent vaccine compositions of the present invention. In particular, adverse reactions were recorded (as show, for example, in Tables 1 and 2 below) and immune responses to each of the antigens contained in the vaccines (as shown, for example, in Tables 3-5 below) were determined. The full liquid and dual chamber formulations were each analyzed in a open, non comparative, randomized study.

350 infants were recruited in a two-arm randomized study to receive a total of four (4) inections of vaccine. The infants were divided into two equal groups. The first group (Group 1) received the multivalent vaccine in the full liquid format. The second group (Group 2) received the multivalent vaccine in a dual chamber format. (As described herein, the "dual chamber" formulation involves the use of an on aluminium salt adsorbed Hepatitis B surface antigen (HBsAg) in the proximal chamber of the syringe, with the remaining components of the vaccine present in a buffered solution in the distal chamber of the syringe).

The vaccination schedule was comprised of an intramuscular injection of the given at two (2), three (3) and four (4) months of age and a final injection at some point between 12 and 14 months of age. Vaccines were administered by intramuscular injection, perpendicular to the skin surface, into the anterolateral aspect of the thigh. Blood samples were taken for antibody titration immediately prior to vaccination and one month after dose 3.

Adverse events were monitored for one month after each immunization and local reaction at the site of injection were recorded within three (3) days after each injection. No vaccine relate serious adverse event was reported during the entire study period.

Local reactions were few and transient and the vaccines were well tolerated.

The IgG response to each component of the multivalent vaccines were compared by standard serological analysis in which the antibody titers following dose 3 were compared to pre-immunization titers. For the most part, there was no significant difference in the results obtained by immunization with the full liquid formulation compared to the dual chamber formulation. Both vaccines provide an excellent and seroprotective immune response against each antigen of their composition. These results are shown in their entirety, on a component by component basis, in Tables 3(a)-(e) below and final results, in terms of seroprotection, are provided in Table 4 below.

In the table 3, the following conventional abbreviations are used. *n* = the number of subject evaluated. GMT = geometric mean titer, and CI = the confidence interval around each GMT value, as determined by standard statistical methodology.

In table 4, the criteria for seroproteciion correspond to the reference commonly admitted by the vaccinology community for each component in terms of expected antibody response obtained after a primary immunisation consisting of three doses given 1 to 2 months apart or after a booster immunisation given about one year after the first immunisation. The criterion for seroprotection for the PT and FHA antigens are 4-fold rise between the pre and post primary series titers and post fourth dose titers. SPR is the seroprotection rate and corresponds to the percentage of subjects fulfilling the criterion of response . GMT has the same meaning as in table 3.

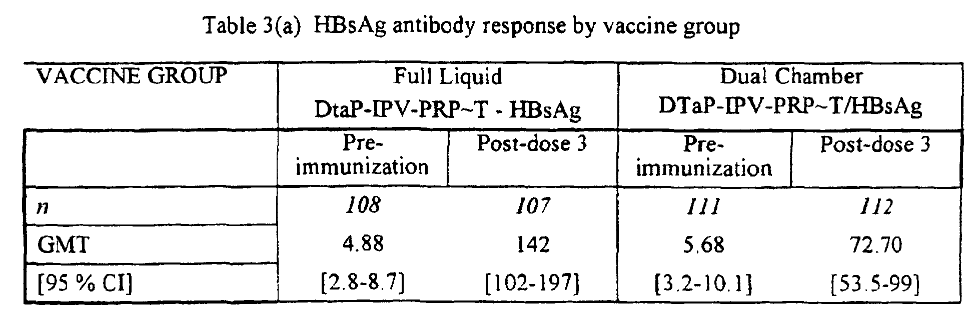

**Table 3(b) PT and FHA (EIA) antibody responses by vaccine group**

| VACCINE GROUP | Full Liquid DtaP-IPV-PRP~T - HBsAg | | Dual Chamber DTaP-IPV-PRP~T/HBsAg | |
|---|---|---|---|---|
| | Pre-immunization | Post-dose 3 | Pre-immunization | Post -dose 3 |
| PT (EIA - EUImL) | | | | |
| *n* | *103* | *108* | *107* | *112* |
| GMT | 2.39 | 53.30 | 2.56 | 61.30 |
| [95%CI] | [1.9-3.0] | [48.0-59.1] | [2.0-3.2] | [55.6-67.6] |
| FHA (EIA - EUImL) | | | | |
| *n* | *102* | *107* | *108* | *112* |
| GMT | 4.73 | 97.70 | 4.88 | 133.0 |
| [95 % CI] | [3.7-6.0] | [86.1-111] | [4.0-6.0] | [119-149] |

**Table 3 (c) Tetanus and diphtheria antibody responses**

| VACCINE GROUP | Full Liquid DTaP-IPV-PRP~T -HBsAg | | Dual Chamber DTaP-IPV-PRP~T/ HBsAg | |
|---|---|---|---|---|
| | Pre-immunization | Post-dose 3 | Pre-immunization | Post-dose 3 |
| Tetanus (EIA - IU/mL) | | | | |
| *n* | *100* | *105* | *101* | *111* |
| GMT | 0.42 | 0.73 | 0.45 | 0.90 |
| [95% CI] | [0.31-0.56] | [0.62-0.90] | [0.34-0.60] | [0.75-1.10] |
| Diphtheria (EIA - | | | | |
| IU/mL) | | | | |
| *n* | *95* | *105* | *95* | *107* |
| GMT | 0.09 | 0.19 | 0.11 | 0.14 |
| [95 % CI] | [0.06-0.14] | [0.15-0.24] | [0.07-0.16] | [0.11-0.18] |

**Table 3(d) Type 1, 2 and 3 poliomyelitis virus neutralising antibody response**

| VACCINE GROUP | Full Liquid DtaP-IPV-PRP~T-HBsAg | | Dual Chamber DTaP-IPV-PRP~T/ HBsAg | |
|---|---|---|---|---|
| | Pre-immunization | Post dose 3 | Pre-immunization | Post dose 3 |
| Polio Type 1 (1/dil.) | | | | |
| *n* | *107* | *108* | *110* | *112* |
| GMT | 63.8 | 254 | 47.4 | 305 |
| [95%CI] | [46.5-87.5] | [196-330] | [35.3-63.6] | [232-403] |
| Polio Type 2 (1/dil.) | | | | |
| *n* | *107* | *108* | *110* | *112* |
| GMT | 85.3 | 115 | 67 | 125 |
| [95 % CI] | [65.6-111] | [87.6-151] | [53.2-84.4] | [93.4-166] |
| Polio Type 3 (1/dil.) | | | | |
| *n* | *107* | *108* | *110* | *112* |
| GMT | 46.9 | 290 | 37.9 | 333 |
| [95 % CI] | [35.6-61.8] | [216-390] | [28.5-50.5] | [252-439] |

**Table 3 (e) PRP antibody response**

| VACCINE GROUP | Full Liquid DtaP-IPV-PRP~T - HBsAg | | Dual Chamber DTaP-IPV-PRP~T/HBsAg | |
|---|---|---|---|---|
| | Pre-immunization | Post-dose 3 | Pre-immunization | Post-dose 3 |
| PRP (RIA - µg/mL) | | | | |
| *n* | *108* | *108* | *110* | *112* |
| GMT | 0.08 | 1.46 | 0.09 | 3.05 |
| [95%CI] | [0.07-0.1] | [1.1-1.9] | [0.08-0.12] | [2.3-4.0] |

**Table 4. Seroprotection for the full Liquid Formulation**

| Antigen | Criteria for seroprotection | n | % of subjects protected |
|---|---|---|---|
| PRP | % ≥ 0.15 µg/ml | 152 | 92.1 |
| HBsAg | % >10 mIU/ml | 151 | 92.7 |
| DT | % >0.01 IU/ml | 144 | 99.3 |
| TT | % >0.01 IU/ml | 147 | 100 |
| Polio type 1 | % titer >5 (Neut.) | 152 | 100 |
| Polio type 2 | % titer >5 (Neut.) | 152 | 100 |
| Polio type 3 | % titer >5 (Neut.) | 152 | 100 |
| PT(EU/mL) | % titer ≥ 4-fold increase | 146 | 87.0 |
| FHA (EU/mL) | % titer ≥ 4-fold increase | 145 | 87.6 |

The safety and immunogenicity results obtained one month after the 2,3,4 months primary series demonstrate the utility of the multivalent vaccines of the present invention. For D, T, and IPV antigens, the immune response was excellent. For the acellular pertussis components, more than eighty-seven percent of these infants showed a four-fold-rise in terms of anti PT anti FHA antibodies.
A good immune response was demonstrated for PRP at the 0.15 µg level, as well as for Hepatitis B, with 92% of seroprotection for both of them. These results are shown in Tables 3 and 4 above.

### Results of Booster Vaccination.

The IgG response to each component of the multivalent vaccines tested were compared by standard serological analysis in which the antibody titers following the final, booster dose given at 12-14 months were compared to pre-booster (post dose 3) titers. These results are shown in their entirety, on a component by component basis, in Tables 5(a)-(e) below and final results, in terms of seroprotection, are provided in Table 6 below. All abbreviations in the following tables are as defined previously for Tables 3(a)-(e) and 4 above.

**Table 5 (a) HBsAg antibody booster response**

| VACCINE GROUP | Full Liquid DtaP-IPV-PRP-T - HBsAg | | Dual Chamber DtaP-IPV-PRP-T/HBsAg | |
|---|---|---|---|---|
| | Pre-Booster | Post-Booster | Pre-Booster | Post-Booster |
| HBsAg (RIA-AUSAB | | | | |
| mIU/mL) | | | | |
| *n* | *125* | *130* | *114* | *119* |
| GMT | 50.4 | 1458 | 37.1 | 530 |
| [95%CI] | [38.1-66.8] | [1056-2013] | [29.1-47.3] | [399-704] |

**Table 5 (b) Pertussis antibody booster responses (PT and FHA)**

| VACCINE GROUP | Full Liquid DtaP-IPV-PRP~T - HBsAg | | Dual Chamber DTaP-IPV-PRP~T/HBsAg | |
|---|---|---|---|---|
| | Pre-Booster | Post-Booster | Pre-Booster | Post-Booster |
| PT (EIA - EUImL) | | | | |
| *n* | *124* | *129* | *112* | *115* |
| GMT | 15.3 | 87.7 | 15 | 101 |
| [95 % CI] | [13.5-17.4] | [79.9-96.2] | [13.1-17.2] | [90.6-112] |
| FHA (EIA - EUImL) | | | | |
| *n* | *123* | *129* | *111* | *115* |
| GMT | 28.9 | 149 | 38.6 | 168 |
| [95%CI] | [25.6-32.6] | [132-168] | [34.4-43.4] | [152-187] |

**Table 5 (c) Tetanus and diphtheria antibody booster responses**

| VACCINE GROUP | Full Liquid DtaP-IPV-PRP~T -HBsAg | | Dual Chamber DTaP-IPV-PRP~T/ HBsAg | |
|---|---|---|---|---|
| | Pre-Booster | Post-Booster | Pre-Booster | Post-Booster |
| Tetanus (EIA - IU/mL) | | | | |
| *n* | *115* | *129* | *105* | *115* |
| GMT | 0.31 | 7.14 | 0.39 | 7.51 |
| [95%CI] | [0.3-0.4] | [6.2-8.1] | [0.3-0.5] | [6.5-8.6] |
| Diphtheria (EIA - | | | | |
| IU/mL) | | | | |
| *n* | *103* | *128* | *102* | *115* |
| GMT | 0.04 | 1.17 | 0.04 | 0.98 |
| [95%CI] | [0.03-0.05] | [0.9-1.4] | [0.03-0.05] | [0.8-1.2] |

**Table 5 (d) Type 1, 2 and 3 poliomyelitis virus antibody booster response**

| VACCINE GROUP | Full Liquid DtaP-IPV-PRP~T-HBsAg | | Dual Chamber DTaP-IPV-PRP~T/ HBsAg | |
|---|---|---|---|---|
| | Pre-Booster | Post-Booster | Pre-Booster | Post-Booster |
| Polio Type 1 (1/dil.) | | | | |
| *n* | *120* | *124* | *104* | *111* |
| GMT | 45.8 | 3113 | 47.3 | 2576 |
| [95%CI] | [35.2-59.6] | [2616-3705] | [34.6-64.7] | [2027-3274] |
| Polio Type 2 (1/dil.) | | | | |
| *n* | *121* | *125* | *107* | *113* |
| GMT | 32.8 | 2496 | 29.2 | 2237 |
| [95%CI] | [25-43] | [2057-3029] | [21-40.6 | [1805-2772] |
| Polio Type 3 (1/dil.) | | | | |
| *n* | *121* | *127* | *109* | *113* |
| GMT | 50.2 | 3938 | 43.6 | 3101 |
| [95%CI] | [37.9-66.4] | [3327-4663] | [31.5-60.4] | [2434-3951] |

**Table 5 (e) PRP antibody booster response**

| VACCINE GROUP | Full Liquid DTaP-IPV-PRP~T - HBsAg | | Dual Chamber DTaP-IPV-PRP~T/HBsAg | |
|---|---|---|---|---|
| | Pre-Booster | Post-Booster | Pre-Booster | Post-Booster |
| PRP (RIA - µg/mL) | | | | |
| *n* | *125* | *129* | *112* | *119* |
| GMT | 0.6 | 28.6 | 0.8 | 31.3 |
| [95%CI] | [0.3-0.6] | [22.3-36.7] | [0.6-1.0] | [24.1-40.8] |

**Table 6 seroprotection for the full Liquid Formulation**

| Components | Criterion for seroprotection | Post-dose 3 | | Post-dose 4 | |
|---|---|---|---|---|---|
| | | SPR(%) | GMT | SPR(%) | GMT |
| PRF | % 0.15 µg/mL | 91.7 | 1.5 | 100.0 | 28.6 |
| HBs | %³ 10mIU/mL | 91.6 | 142.0 | 98.5 | 1458.0 |
| Diphteria | %³0.01 IU/mL | 99.0 | 0.2 | 100.0 | 1.2 |
| Tetanus | %³0.01 IU/mL | 100.0 | 0.7 | 100.0 | 7.1 |
| Polio 1 | % titre³ 5 (Neut) | 100.0 | 254.0 | 100.0 | 3113.0 |
| Polio 2 | % titre³ 5 (Neut) | 100.0 | 115.0 | 100.0 | 2496.0 |
| Polio 3 | % titre³ 5 (Neut) | 100.0 | 290.0 | 100.0 | 3938.0 |
| PT | % 4-fold rise (EU/mL) | 88.3 | 53.3 | 69.4 | 87.7 |
| FHA | % 4-fold rise (EU/mL) | 87.7 | 97.7 | 69.2 | 149.0 |

For all antigens, we observed an excellent booster effect induced by the fourth dose confirming the presence of an excellent immune memory induced by this accelerated 2-3-4 month primary series.These results are shown in Tables 5 and 6 above.

The compositions of the invention provide a seroprotection against each disease.

### SUMMARY OF THE DISCLOSURE

The preparation of numerous multivalent vaccines are described clearly above. Extensive clinical trials described above clearly demonstrate that the multivalent immunological compositions of the present invention are safe and efficacious for conferring protection against a broad range of pathogens.

These results are surprising insofar as mixtures of numerous vaccine components may have been expected to contribute to well-recognized phenomena of antigenic competition or interference, whereby certain vaccine components which would be capable of conferring seroprotection when introduced individually into an immunocompetent host become less effective when introduced in combination with other antigens. Thus, the vaccines of the present invention simplify the immunization process and greatly minimize the number of separate immunizations needed to protect pediatric patients from infection with *Bordatella pertussis*, *Corynebacterium diphtheriae, Clostridium tetanae*, *Haemophilus influenzae*, poliovirus and Hepatitis b virus.

### REFERENCES

1. Muller, A.S. Leeuwenburg, J. and Pratt, D.S. (1986) Pertussis: epidemiology and control. *Bull WHO* 64: 321-331.
2. Fine, P.E.M. and Clarkson, J.A. (1984). Distribution of immunity to pertussis in the population of England and Wales. *J. Hyg*. 92: 21-26
3. Mortimer, E.A. Jr. (1990). Pertussis and its prevention: a family affair. *J*. *Infect. Dis.* 161: 473-479.
4. Addiss, D.G., Davis, I.P., Meade, B.D., Burstyn, D.G. Meissner, M., Zastrow, J.A., Berg, J.L., Drinka, P., and Phillips, R. (1991). A pertussis outbreak in a Wisconsin nursing home. *J*. *Infect. Dis.* 164: 704-710.
5. Halperin, S.A. and Marrie, T.J. (1991). Pertussis encephalopthy in an adult: case report and review. *Rev. Infect. Dis.* 13: 1043-1047.
6. Onorato, I.M., Wassilak, S.G. and Meade, B. (1992). Efficacy of whole-cell pertussis vaccine in preschool children in the United States. *JAMA* 267: 2745-2749.
7. Miller, D.L., Ross, E.M., Alderslade, R., Bellman, M.H., and Brawson, N.S.B. (1981). Pertussis immunization and serious acute neurological illness in children: *Brit Med*. *J*. 282: 1595-1599.
8. Tamura, M., Nogimori, K., Murai, S., Yajima, M., Ito, K., Katada, T., Ui, M., and Ishii, S. (1982). Subunit structure of islet-activating protein. pertussis toxin, in conformity with the A-B model. *Biochemistry* 21: 5516-5522.
9. Tuomanen, E. and Weiss, A. (1985). Characterization of two adhesins of *Bordetella pertussis* for human ciliated respiratory epithelial cells. *J. Infect. Dis.* 152: 118-125.
10. Friedman, R-L., Nordensson, K., Wilson, L., Akporiaye, E.T., and Yocum, D.E. (1992). Uptake and intracellular survival of *Bordetella pertussis* in human macrophages. *Infect. Immun.* 60: 4578-4585.
11. Pittman, M (1979). Pertussis toxin: the cause of the harmful effects and prolonged immunity of whooping cough. A hypothesis. *Rev. Infect. Dis.*1: 401-402.
12. Granstrom, M. and Granstrom G. (1993). Serological correlates in whooping cough. *Vaccine* 11: 445-448.
13. Gearing, A.J.H., Bird, C.R., Redhead, K., and Thomas, M. (1989). Human cellular immune responses to *Bordetella pertussis* infection. *FEMS Microbial. Immunol. 47:* 205-212.
14. Thomas, M.G., Redhead, K., and Lambert, H.P. (1989). Human serum antibody responses to *Bordetella pertussis* infection and pertussis vaccination. *J*. *Infect.* Dis. 159: 211-218.
15. Thomas, M.G., Ashworth, L.A.E., Miller, E., and Lambert, H.P. (1989). Serum IgG, IgA, and IgM responses to pertussis toxin, filamentous haemagglutonin, and agglutinogens 2 and 3 after infection with *Bordetella pertussis* and immunization with whole-cell pertussis vaccine. *J. Infect*. *Dis*. 160: 838-845.
16. Tomoda, T., Ogura, H., and Kurashige, T. (1991). Immune responses to Bordetella pertussis infection and vaccination. *J*. *Infect. Dis.* 163: 559-563.
17. Petersen, J.W., Ibsen. P.H., Haslov, K., Capiau, C., and Heron, I. (1992). Proliferative responses and gamma interferon and tumor necrosis factor production by lymphocytes isolated from trachcobroncheal lymph nodes and spleens of mice aerosol infected with *Bordetella pertussis*. *Infect*. *Immun.* 60: 4563-4570.
18. Englund, J.A., Reed, G.F., Edwards, K.M., Decker, D., Pichichero, M.E., Ronnels, M.B., Steinhoff, M.C., Anderson, E.L., Meade, B.D., Deloria, M.A., and the NIAID Acellular Pertussis Vaccine Group. (1992). Effect of transplacental antibody and dvelopment of pertussis toxin (PI) and filamentous haemagglutonin (FHA) antibody after acellular (AC) and whole cell (WC) pertussis vaccines in infants. *Pediat. Res.* 31: 91A.
19. Oda, M., Cowell, J.L., Burstyn, D.G., Thaib, S., and Manclark, C.R. (1985). Antibodies to *Bordetella pertussis* in human colostrum and their protective activity against aerosol infection of mice. *Infect. Immun.* 47: 441-445.
20. Petersen, J.W., P.H. Bentzon, M. W., Capiau, C., and Heron, I. (1991). The cell mediated and humoral immune response to vaccination with acellular and whole cell pertussis vaccine in adult human. *FEMSMicrobiol Lett.* 76: 279-288.
21. Oda, M., Cowell. J.L., Burstyn, D.G., and Manclark, C.R. (1984). Protective activities of the filamentous haemagglutonin and the lymphocytosis-promoting factor of *Bordetella pertussis* in mice. *J. Infect. Dis.* 150: 823-833.
22. Sato, H., Ito, A., Chiba, J. and Sato. Y. (1984). Monoclonal antibody against pertussis toxin: effect on toxin activity and pertussis infections. *Infect. Immun.* 46:422-428.
23. Sato, H. and Sato, Y. (1990). Protective activities in mice of monoclonal antibodies against pertussis toxin. *Infect. Immun.* 58:3369-3374.
24. Weiss, A.A. and Hewlett, E.L. (1986). Virulence factors of *Bordetella pertussis. Ann. Rev. Microbiol* 40: 661-668.
25. Munoz, J.J. (1988). Action of pertussigen (pertussis toxin) on the host immune system. In: *Pathogenesis and Immunity in Pertussis.* A.C. Wardlaw and R. Parton, eds., John Wiley & Sons Ltd., Toronto. pp. 211-229.
26. Watkins, P.A., Burns, D.L., Kanaho, Y., Liu, T-Y., Hewlett E.L., and Moss, J. (1985) ADP-ribosylation of transducin by pertussis toxin. *J. Biol. Chem.* 260: 13478-13482.
27. Bums, D.L., Kenimer, J.G., and Manclark, C.R. (1987). Role of the A subunit of periussis toxin in alteration of Chinese hamster ovary cell morphology. *Infect. Immun.* 55: 24-28.
28. Munoz, J.J., Arai, H., and Cole, R.L. (1981). Mouse-protecting and histaminesensitizing activities of pertussigen and fimbrial hemagglutinins from *Bordetella pertussis. Infect*. *Immun*. 32: 243-250.
29. Relman, D.A., Domenighini, M., Tuomanen, E., Rappuoli, R., and Falkow, S. (1989). Filamentous haemagglutinin of Bordetella pertussis: nucleotide sequence and crucial role inadherence. *Proc. Natl. Acad Sci. USA* 86: 2637-2641.
30. Di Tommaso, A., Domenighini, M., Bugnoli, M., Tagliabuc, A., Rappuoli, R., and De Magistris, M.T. (1991). Identification of subregions of *Bordelelia* pertussis filamentous haemagglutonin that stimulate human T-cell responses. *Infect. Immun.* 59: 3313-3315.
31. Tomoda, T., Ogura, H., and Kurashige, T. (1992). The longevity of the immune response to filamentous haemagglutonin and pertussis toxin in patients with pertussis in a semiclosed community. *J*. *Infect. Dis.* 166: 908-910.
32. Edwards, K.M., Meade, B.D., Decker, M.D., Reed, G.F., Rennels, M.B., Steinhoff, M.C., Anderson, E.L., Englund, J.A., Pichichero, M.E., Deloria, M.A., Deforest, A., and the NIAID Acellular Pertussis Vaccine Study Group (1992). Comparison of thirteen acellular pertussis vaccines: serological response. *Pediatr*. *Res.* 31: 91A.
33. Kimura, A., Mountzoutos, K.T., Relman, D.A., Falkow, S., and Cowell, J.L. (1990a). *Bordetella pertussis* filamentous haemagglutonin: evaluation as a protective antigen and colonization factor in a mouse respiratory infection model. *Infect*. *Immun*. 58: 7-16.
34. Shahin, R.D., Amsbaugh, D.F., and Leef, M.F. (1992). Mucosal immunization with filamentous haemagglutonin protects against *Bordetella pertussis* respiratory infection. *Infect. Immun.* 60:1482-1488.
35. Montaraz, J.A., Novotny, P.. and Ivanyl, J. (1985). Identification of a 68-kilodalton protective protein antigen from *Bordetella bronchiseptica. Infect. Immun.* 161: 581-582.
36. Leininger, E., Roberts, M., Kenimer, J.G., Charles, I.G., Fairweather, M., Novotny, P., and Brennan, M.J. (1991). Pertactin, and Arg-Gly-Asp-containing *Bordetella pertussis* surface protein that promotes adherence of mammalian cells. *Proc. Natl. Acad Sci. USA* 88: 345-349.
37. De Magistris, T., Romano, M., Nuti, S., Rappuoli, R. and Tagliabue, A. (1988). Dissecting human T responses against *Bordetella* species *J*. *Exp. Med.* 168: 1351-1362.
38. Seddon, P.C., Novotny, P., Hall, C.A., and Smith, C.S. (1990). Systemic and mucosal antibody response to *Bordetella pertussis* antigens in children with whooping cough. *Serodiagnosis Immunother. Inf*. *Dis*. 3: 337-343.
39. Podda, A., Nencioni, L., Marsili, I., Peppoloni, S., Volpini, G., Donati, D., Di Tommaso, A., De Magistris, M.T., and Rappuoli, R. (1991). Phase I clinical trial of an acellular pertussis vaccine composed of genetically detoxified pertussis toxin combined with FHA and 69kDa. *Vaccine* 9: 741-745.
40. Roberts, M., Tite, J.P., Fairweather, N.F., Dougan, G. and Charles, I.G. (1992). Recombinant P.69/pertactin: immunogenicity and protection of mice against *Bordetella pertussis* infection. *Vaccine* 10: 43-48.
41. Novotny, P., Chubb, A.P., Cownley, K., and Charles, I.G. (1991). Biological and protective properties of the 69kDa outer membrane protein of *Bordetella pertussis:* a novel formulation for an acellular vaccine. *J. Infect. Dis.* 164: 114-122.
42. Shahin, R.D., Brennan, M.J., Li. Z.M., Meade, B.D., and Manclark, C.R. (1990). Characterization of the protective capacity and immunogenicity of the 69kD outer membrane protein of *Bordetella pertussis*. *J*. *Exp*. *Med* 171: 63-73.
43. Robinson, A., Irons, L.I., and Ashworth, L.A.E. (1985). Pertussis vaccine: present status and future prospects *Vaccine* 3: 11-22.
44. Robinson, A., Ashworth, L.A.E. Baskerville, A., and Irons, L.I. (1985). Protection against intranasal infection of mice with *Bordetella pertussis*. *Develop*. *Biol*. *Stand 61:* 165-172.
45. Robinson, A., Gorrige, A.R., Funnell, S.G.P., and Fernandez, M. (1989). Serospecific protection of mice against in infection with *Bordetellapertussis. Vaccine* 7: 321 -324.
46. Sata, Y., Kimura, M., and Fukumi, H. (1984). Development of a pertussis component vaccine in Japan. *Lancet* i: 122-126.
47. Kimura, M. (1991). Japanese clinical experiences with acellular pertussis vaccines. *Develop. Biol. Standard.* 73: 5-9.
48. Ad Hoc Group for the Study of Pertussis Vaccines (1988). Placebo-controlled trial of two acellular vaccines in Sweden-protective efficacy and adverse effects. *Lancet i:* 955-960.
49. Olin, P., Storsaeter, J., and Romanus, V. (1989). The efficacy of acellular pertussis vaccine. *JAMA* 261: 560.
50. Storsaeter, J., Hallander, H., Farrington, C.P., Olin, P., Moliby, R., and Miller, E. (1990). Secondary analyses of the efficacy of two acellular pertussis vaccines evaluated in a Swedish phase III trial. *Vaccine* 8: 457-462.
51. Storsaeter, J., and Olin, P. (1992). Relative efficacy of two acellular pertussis vaccines during three years of passive surveillance. *Vaccine* 10: 142-144.
52. Tan, L.U.T., Fahim R.E.F., Jackson, G., Phillips, K., Wah, P., Alkema, D., Zobrist, G., Herbert, A., Boux. L, Chong, P., Harjee, N., Klein, M., and Vose, J. (1991). A novel process for preparing an acellular pertussis vaccine composed of non-pyrogenic toxoids of pertussis toxin and filamentous haemagglutonin. *Molec*. *Immunol.* 28: 251-255.
53. Sekura, R.D., Zhang, Y., Roberson, R., Action, B., Trollfors, B., Tolson, N., Siloach, J., Bryla, D., Muir-Nash, J., Koeller, D., Schneerson, R., and Robbins, J.B. (1988). Clinical, metabolic, and antibody responses of adult volunteers to an investigation vaccine composed of pertussis toxin inactivated by hydrogen peroxide. *J. Pediatr.* 113: 807-813.
54. Winberry, L., Walker, R., Cohen, N., Todd, C., Sentissi, A., and Siber, G. (1988). Evaluation of a new method for inactivating pertussis toxin with tetranitromethane. International *Workshop on Bordetella pertussis,* Rocky Mountain Laboratories, Hamilton, Montana.
55. Sekura, R.D. et al. (1993), *J*. *Biol. Chem*. 258: 14647-14651.
56. Irons, L.I. and MacLennan, A.P.. (1979). Isolation of the lymphocytosis promoting factor-haemagglutinin of Bordetella pertussis by affinity chromatography. *Biochem*. *Biophys. Acta* 580: 175-185.
57. Munoz, J.J., Arai, H., Bergman, R.K., and Sadowski, P.L. (1981). Biological activities of crystalline pertussigen from Bordetella pertussis. (1981). *Infect. Immun.* 33: 820-826.
58. Cowell, J.L. et al. (1980), Seminar on Infectious Diseases 4: 371-379.
59. Selmer, J.C., Larsen, F.S., Hertz, J.B., Parton, R. (1984). Purification and partial characterization of filamentous haemagglutinin from Bordetella pertussis using monoclonal antibodies. *Acta Path. Microbial. Immunol. Scand.* Sect. C, 92: 279-284.
60. Lockhoff, O. (1991). Glycolipids as Immunomodulators: Synthesis and Properties, *Chem.Int. Ed. Engl.* 30: 1611-1620.
61. Nixon-George, A., Moran, T., Dionne, G., Penney, C.L., Lafleur, D., and Bona, C.A. (1990). The adjuvant effect of stearyl tyrosine on a recombinant subunit hepatitis B surface antigen. *J. Immunol.* 144: 4798-4802.
62. Siber, G.R., Thakrar, N., Yancey, B.A., Herzog, L., Todd, C., Cohen, N., Sekura, R.D., Lowe, C.U. (1991). Safety and immunogenicity of hydrogen peroxide-inactivated pertussis toxoid in 18-month-old children. *Vaccine* 9: 735-740.
63. Siber, G., Winberry, L., Todd, C., Samore, M., Sentissi, A., and Cohen, N. (1988). Safety and immunogenicity in adults of pertussis toxoid inactivated with tetronitromethane. In: *International Workshop on Bordetella pertussis,* Rocky Mountain Laboratories, Hamilton, Montana.
64. Edwards, K.M., Bradley, R.B., Decker, M.D. , Palmer, P.S., Van Savage, J., Taylor, J.C., Dupont, W.D., Hager, C.C., and Wright, P.F. (1989). Evaluation of a new highly purified pertussis vaccine in infants and children. *J. Infect. Dis.* 160: 832-837.
65. Rutter, D.A., Ashworth, L.A.E., Day, Aj., Funnell, S., Lovell, F., and Robinson, A. (1988). Trial of new acellular pertussis vaccine in healthy adult volunteers. *Vaccine 6:* 29-32.
66. Blumberg, D.A., Mink, C.A.M., Cherry, J.D., Johnson, C., Garber, R., Plotkin, S.A.. Watson, B., Ballanco, G.A., Daum R.S., Sullivan B., Townsend, T.R. Brayton, J., Gooch, W.M., Nelson, D.B., Congeni, B.L., Prober, C.G., Hackell, J.G., Dekker, C.L., Christenson, P.D., and the APDT Vaccine Study Group (1991). Comparison of acellular and whole cell pertussis-component diphtheria-tetanus-pertussis vaccines in infants. *J. Pediatr*. 119: 194-204.
67. Englund, J.A., Glezen, W.P. and Barreto, L. (1992). Controlled study of a new five-component acellular pertussis vaccine in adults in young children. *J*. *Inf*. *Dis*. 166: 1436-1441.
68. Zealey, G., Loosmore, S., Yacoob, R., and Klein, M., Vaccine Research, Vol. 1, pp. 413-427.
69. Baker, J.D., Halperin, S.A., Edwards, K., Miller, B., Decker, M. and Stephens, D. (1992). Antibody response to Bordetella pertussis antigens after immunization with American and Canadian whole cell vaccines *J*. *Pediatr.* 121: 523-527.
70. Halperin S.A., Eastwood, B.J. and Langley, J.M. Immune responses to pertussis vaccines concurrently administered with viral vaccines. *Ann NY Acad Sci.* 1995, 754: 89-96.
71. Halperin S.A., Langley, J.M. and Eastwood, B.J. (1996). Effect of inactivated poliovirus vaccine on the antibody response to Bordetella pertussis antigens when combined with diphtheria-pertussis-tetanus vaccine. *Clin. Infect. Dis.* 22: 59-62.
72. Ferreccio, C., Clemens, J., Avendano, A., Horwitz, I., Flores, C., Avila, L., Cayazzo, M., Fritzell, B., Cadoz, M. and Levine, M.. (1991). The clinical and immunogenic response of Chilean infants to Haemophilus influenzae type b polysaccharide tetanus protein conjugate vaccine coadministered in the same syringe with diptheria-tetanus toxoide-penussis vaccine at two, four and six months of age. *Pediatr. Infect. Dis. J.* 10: 761-771.
73. Clemens, J.D., Ferreccio, C., Levine, M.M., Horwitz, I., Rao, M.R., Edwards, K.M. and Fritzell, B. (1992). Impact of Haemophilus influenzae type b polysaccharide-tetanus protein conjugate vaccine on responses to concurrently administered diphetheria-tetanus pertussis vaccine. *JAMA* 267: 673-8.
74. Scheifele D. Barreto L. Meekison W. *et al.* (1993). Can Haemophilus influenazae vaccine be combined with diptheria and tetanus toxoids. *Can. Med Assoc. J.* 149: 1105-16.
75. Gold, R., Scheifele, D., Barreto, L., Wiltsey, S., Bjornson, G., Meekison, W., Guasparini, R. and Medd, L. (1994). Safety and immunogenicity of Haemophilus influnzae vaccine (tetanus toxoid conjugate) administered concurrently or combined with diptheria and tetanus toxoids, pertussis vaccine and inactivated poliomyelitis vaccine to healthy infants at two, four and six months of age. *Pediatr. Infect. Dis J.* 13: 348-55.
76. Shinefield, H. Black, S., Ray, P., Lewis, E. and Fireman, B., Hohenboken, Hackell, JG. Safety of combined acellular pertussis vaccine in infants (abstract no. G72). In: Program and Abstracts of the 35th Interscience Conference on Antimicrobiols and Chemotherapy. Washington, DC; American Society of Microbiology 195:171.
77. Greenberg, D.P., Wong, V.K., Partridge, S., Howe, B.J., Fing, J. and Ward, JL. Evaluation of a new combination vaccine that incorporates diphtheria-tetanus-acellular pertussis, hepatitis b, and *Haemophilus influenzae* type b conjugate vaccines (Abstract no G70). In Program and Abstracts of the 35th Interscience Conference on Antimicrobiols and Chemotherapy. Washingotn, DC; American Society of Microbiology 1995: 170.
78. Wassilak, S.G.F. and Orenstein, W.A., Tetanus, In Plotkin SA, Mortimer EA, Jr., eds, Vaccines, W.B. Saunders Company, Philadelphia, 1988; 45-73.
79. Benenson, A.S. (Editor), The Control of Communicable Diseases in Man 15th Edition. 1990, Diphtheria; 138-142, Tetanus: 430-435; Pertussis: 318-322.
80. Mortimer.,E.A., Jr., Diphtheria Toxoid, In Plotkin SA, Mortimer EA, Jr., eds, Vaccines, WB Saunders Company, Philadelphia, 1988; 31-44.
81. Varughese, P. (1986). *Haemophilus influenzae* infection in Canada, 1969-1985. *Can. Dis. Wkly Rep.* 12: 37-43.
82. Schelfele, D., Gold, R., Law, B., *et al.* (1993). Decline in Haemophilus Influenzae type b invasive infections at five Canadian pediatric centres. *Can. Commun. Dis. Rep.* 19: 88-91.
83. Scheifele D., Barreto L., Meekison W. et al.: Can *Haemophilus influenzae* type b-tetanus toxoid conjugate vaccine be combined with diphtheria toxoid-pertussis vaccine-tetanus toxoid? Can Med Assoc J 1993, 149: 1105-1112.
84. Gold R, Scheifele D, Baretto L et al.: Safety and Immunogenicity of a *Haemophilus Influenzae* type b vaccine (tetanus toxoid conjugate) administered concurrently or combined with diphtheria and tetanus toxoids, pertussis vaccine and inactivated poliomyelitis vaccines to healthy infants at two, four six months of age. Pediatric Infectious Diseases Journal 1994; 13: 348-55.
85. Scheifele D, Gold R, et al. Canada Communicable Diaease Report 22-3, F1-F3 Feb. 1, 1996.
86. Amir J et al. Vaccine 1997 15: 149

## Claims

1. A method for preparing a vaccine composition comprising:
(a) pertussis toxoid and filamentous haemagglutinin in purified form,
(b) tetanus toxoid,
(c) diphtheria toxoid,
(d) inactivated polio virus,
(e) an *Haemophilus influenzae* type B polysaccharide conjugate,
(f) an Hepatitis B surface antigen and
(g) an aluminium salt,
said method comprising the following steps:
(1) preparing an acidified aluminium hydroxide gel suspension at room temperature,
(2) adding successively diphtheria toxoid and tetanus toxoid to the gel suspension;
(3) adding pertussis toxoid and filamentous haemagglutinin, each adsorbed separately onto aluminium salts, to the mixture obtained in (2), followed by a at least 30 minute agitation and a further overnight rest;
(4) adding to the mixture obtained in (3) carbonate buffers in medium 199 and adjusting the pH to about 7,0 to 7,2;
(5) introducing inactivated poliovirus into the mixture obtained in (4) and then adjusting the pH to a value ranging from 6,8 to 7,0 ;
(6) adding Hepatitis B surface antigen previously adsorbed onto aluminium salts, to the mixture obtained in (5), followed by a at least 30 minute agitation; and
(7) adding a solution of an *Haemophilus influenzae* type B polysaccharide conjugate previously prepared in a phosphate buffer and a Tris-sucrose buffer to the mixture obtained in (6), followed by a at least 30 minute agitation.

2. A method for preparing a vaccine composition comprising:
(a) pertussis toxoid and filamentous haemagglutinin in purified form,
(b) tetanus toxoid,
(c) diphtheria toxoid,
(d) inactivated polio virus,
(e) an *Haemophilus influenzae* type B polysaccharide conjugate,
(f) an Hepatitis B surface antigen and
(g) an aluminium salt
said method comprising the following steps:
(1) preparing an acidified aluminium hydroxide gel suspension at room temperature ;
(2) adding diphtheria toxoid and tetanus toxoid to the gel suspension ;
(3) adding pertussis toxoid and filamentous haemagglutinin, each adsorbed separately onto aluminium salts, to the mixture obtained in (2), followed by a at least 30 minute agitation and a further overnight rest;
(4) adding to the mixture obtained in (3) carbonate buffers in medium 199 and adjusting the pH to about 7,0 to 7,2.
(5) introducing inactivated poliovirus into the mixture obtained in (4) and then adjusting the pH to a value ranging from 6,8 to 7,0 ;
(6) adding a solution of an *Haemophilus Influenzae* type B polysaccharide conjugate prepared in a phosphate buffer and a Tris-sucrose buffer to the mixture obtained in (5) followed by a at least 30 minute agitation ;
(7) disposing 0,5 ml of the mixture obtained in (6) in the distal chamber of a 1ml by-pass syringe; and
(8) adding 0,5 ml of Hepatitis B surface antigen previously adsorbed onto aluminium salts, in the proximal chamber of the by-pass syringe used in step (7).

## Patentansprüche

1. Verfahren zur Herstellung einer Impfstoffzusammensetzung umfassend:
(a) Pertussistoxoid und filamentöses Hämagglutinin in gereinigter Form,
(b) Tetanustoxoid,
(c) Diphtherietoxoid,
(d) inaktiviertes Poliovirus,
(e) ein *Haemophilus-influenzae*-Typ-B-Polysaccharidkonjugat.
(f) ein Hepatitis-B-Oberflächenantigen und
(g) ein Aluminiumsalz,
wobei das Verfahren die folgenden Schritte umfasst:
(1) Herstellen einer angesäuerten Aluminiumhydroxidgelsuspension bei Raumtemperatur;
(2) stufenweise Hinzugabe von Diphtherietoxoid und Tetanustoxoid zu der Gelsuspension;
(3) Hinzugabe von Pertussistoxoid und filamentösem Hämagglutinin, wobei jedes voneinander getrennt auf Aluminiumsalzen adsorbiert wurde, zu der in (2) erhaltenen Mischung, gefolgt von mindestens 30 Minuten Rühren und einem weiteren Stehenlassen über Nacht;
(4) Hinzugabe von Carbonatpuffern in Medium 199 zu der in (3) erhaltenen Mischung und Einstellen des pH-Wertes auf ungefähr 7,0 bis 7,2;
(5) Einbringen von inaktiviertem Poliovirus in die in (4) erhaltene Mischung und dann Einstellen des pH-Wertes auf einen Wert zwischen 6,8 und 7,0;
(6) Hinzugabe von Hepatitis-B-Oberflächenantigen, welches vorher auf Aluminiumsalzen adsorbiert wurde, zu der in (5) erhaltenen Mischung, gefolgt von mindestens 30 Minuten Rühren; und
(7) Hinzugabe einer Lösung eines Haemophilus-influenzae-Typ-B-Polysaccharidkonjugates, welche vorher in einem Phosphatpuffer und einem Tris-Saccharosepuffer hergestellt wurde, zu der in (6) erhaltenen Mischung, gefolgt von mindestens 30 Minuten Rühren.

2. Verfahren zur Herstellung einer Impfstoffzusammensetzung umfassend:
(a) Pertussistoxoid und filamentöses Hämagglutinin in gereinigter Form,
(b) Tetanustoxoid,
(c) Diphtherietoxoid,
(d) inaktiviertes Poliovirus,
(e) ein *Haemophilus-influenzae*-Typ-B-Polysaccharidkonjugat,
(f) ein Hepatitis-B-Oberflächenantigen und
(g) ein Aluminiumsalz,
wobei das Verfahren die folgenden Schritte umfasst:
(1) Herstellen einer angesäuerten Aluminiumhydroxidgelsuspension bei Raumtemperatur;
(2) Hinzugabe von Diphtherietoxoid und Tetanustoxoid zu der Gelsuspension;
(3) Hinzugabe von Pertussistoxoid und filamentösem Hämagglutinin, wobei jedes voneinander getrennt auf Aluminiumsalzen adsorbiert wurde, zu der in (2) erhaltenen Mischung, gefolgt von mindestens 30 Minuten Rühren und einem weiteren Stehenlassen über Nacht;
(4) Hinzugabe von Carbonatpuffern in Medium 199 zu der in (3) erhaltenen Mischung und Einstellen des pH-Wertes auf ungefähr 7,0 bis 7,2;
(5) Einbringen von inaktiviertem Poliovirus in die in (4) erhaltene Mischung, und dann Einstellen des pH-Wertes auf einen Wert zwischen 6,8 und 7,0;
(6) Hinzugabe einer Lösung eines Haemophilus-influenzae-Typ-B-Polysaccharidkonjugates, welche in einem Phosphatpuffer und einem Tris-Saccharosepuffer hergestellt wurde, zu der in (5) erhaltenen Mischung, gefolgt von mindestens 30 Minuten Rühren;
(7) Einfüllen von 0,5 ml der in (6) erhaltenen Mischung in die distale Kammer einer 1ml-Bypassspritze; und
(8) Hinzugabe von 0.5 ml Hepatitis-B-Oberflächenantigen, welches vorher auf Aluminiumsalzen adsorbiert wurde, in die proximale Kammer der in Schritt (7) verwendeten Bypassspritze.

## Revendications

1. Procédé de préparation d'une composition vaccinale comprenant :
(a) de l'anatoxine et de l'hémagglutinine filamenteuse coquelucheuses sous forme purifiée,
(b) de l'anatoxine tétanique,
(c) de l'anatoxine diphtérique,
(d) du poliovirus inactivé,
(e) un conjugué polysaccharidique *d'Haemophilus influenzae* de type B,
(f) un antigène de surface de l'hépatite B et
(g) un sel d'aluminium,
ledit procédé comprenant les étapes suivantes :
(1) la préparation d'une suspension de gel d'hydroxyde d'aluminium acidifié à température ambiante ;
(2) l'addition successive d'anatoxine diphtérique et d'anatoxine tétanique à la suspension de gel ;
(3) l'addition d'anatoxine et d'hémagglutinine filamenteuse coquelucheuses, chacune adsorbée séparément sur des sels d'aluminium, au mélange obtenu en (2), suivie d'une agitation d'au moins 30 minutes et en outre d'un repos pendant une nuit ;
(4) l'addition au mélange obtenu en (3) de tampons carbonates dans du milieu 199 et l'ajustement du pH à environ 7,0 à 7,2 ;
(5) l'introduction de poliovirus inactivé dans le mélange obtenu en (4) et ensuite l'ajustement du pH à une valeur située dans la plage de 6,8 à 7,0 ;
(6) l'addition d'antigène de surface de l'hépatite B précédemment adsorbé sur des sels d'aluminium, au mélange obtenu en (5), suivie d'une agitation d'au moins 30 minutes ; et
(7) l'addition d'une solution d'un conjugué polysaccharidique *d'Haemophilus influenzae* de type B précédemment préparée dans un tampon phosphate et un tampon Tris-saccharose, au mélange obtenu en (6), suivie d'une agitation d'au moins 30 minutes.

2. Procédé de préparation d'une composition vaccinale comprenant :
(a) de l'anatoxine et de l'hémagglutinine filamenteuse coquelucheuses sous forme purifiée,
(b) de l'anatoxine tétanique,
(c) de l'anatoxine diphtérique,
(d) du poliovirus inactivé,
(e) un conjugué polysaccharidique *d'Haemophilus influenzae* de type B,
(f) un antigène de surface de l'hépatite B et
(g) un sel d'aluminium,
ledit procédé comprenant les étapes suivantes :
(1) la préparation d'une suspension de gel d'hydroxyde d'aluminium acidifié à température ambiante ;
(2) l'addition d'anatoxine diphtérique et d'anatoxine tétanique à la suspension de gel ;
(3) l'addition d'anatoxine et de d'hémagglutinine filamenteuse coquelucheuses, chacune adsorbée séparément sur des sels d'aluminium, au mélange obtenu en (2), suivie d'une agitation d'au moins 30 minutes et en outre d'un repos pendant une nuit ;
(4) l'addition au mélange obtenu en (3) de tampons carbonates dans du milieu 199 et l'ajustement du pH à environ 7,0 à 7,2 ;
(5) l'introduction de poliovirus inactivé dans le mélange obtenu en (4) et ensuite l'ajustement du pH à une valeur située dans la plage de 6,8 à 7,0 ;
(6) l'addition d'une solution d'un conjugué polysaccharidique *d'Haemophilus influenzae* de type B précédemment préparée dans un tampon phosphate et un tampon Tris-saccharose, au mélange obtenu en (5), suivie d'une agitation d'au moins 30 minutes ;
(7) le dépôt de 0,5 ml du mélange obtenu en (6) dans la chambre distale d'une seringue à double compartiment de 1 ml ; et
(8) l'addition de 0,5 ml de l'antigène de surface de l'hépatite B précédemment adsorbé sur des sels d'aluminium, dans la chambre proximale de la seringue à double compartiment utilisée dans l'étape (7).
